# EUROPÄISCHE PATENTSCHRIFT

(11) **EP 0 814 803 B1**
(45) Veröffentlichungstag und Bekanntmachung des Hinweises auf die Patenterteilung: **11.10.2006**
(21) Anmeldenummer: 96906768.5
(22) Anmeldetag: 15.03.1996
(51) Int. Cl.: A61K 31/365, A61K 31/135, A61K 31/40, A61K 31/445, A61K 31/435, A61K 31/41, A61K 31/55, A61K 31/565

(54) **Verwendung von steroidalen Estrogenrezeptorantagonisten zur männlichen Fertilitätskontrolle**
Use of steroidal estrogen receptor antagonists as male contraceptives
utilisation d'antagonistes steroidiens du receptor d'estrogen comme contraceptifs masculins

(30) Priorität: 16.03.1995 DE 19510862
(43) Veröffentlichungstag der Anmeldung: 07.01.1998
(73) Patentinhaber: Schering Aktiengesellschaft, 13353 Berlin (DE)
(72) Erfinder: HABENICHT, Ursula-Friederike, D-14167 Berlin (DE)
(86) Internationale Anmeldenummer: PCT/EP1996/001191
(87) Internationale Veröffentlichungsnummer: WO 1996/028154

(56) Entgegenhaltungen:
- EP-A- 0 346 014
- EP-A- 0 488 383
- WO-A-93/10741
- WO-A-93/13123
- DE-A- 3 213 328
- J REPROD FERTIL, NOV 1993, 99 (2) P395-402, ENGLAND, XP000600346 GILL-SHARMA MK ET AL: "Effects of tamoxifen on the fertility of male rats."
- TOXICOLOGY AND APPLIED PHARMACOLOGY, Bd. 91, Nr. 2, 1987, Seiten 235-245, XP000645922 HIRSCH, K.S. ET AL: "Inhibition of central nervous system aromatase activity: A mechanism for Fenarimol-induced infertility in the male rat"

## Beschreibung

Die vorliegende Erfindung betrifft die Verwendung von Antiestrogenen zur Herstellung von Arzneimitteln zur Kontrolle der männlichen Fertilität.

Antiestrogene umfassen im engeren Sinn die Substanzklasse derjenigen Verbindungen, die Estrogene von ihren jeweiligen Rezeptoren verdrängen können (Estrogenrezeptorantagonisten) und im weiteren Sinn auch die Verbindungen, die die Synthese von Estrogenen aus ihren metabolischen Vorstufen im Organismus -androgene Verbindungen mit 3-Keto-4-en-Steroidstruktur -durch Inhibierung des Enzyms Aromatase verhindern (Aromatasehemmer). Zu diesen beiden Gruppen, die letztlich die biologische Wirkung von Estrogenen hemmen, gehören jeweils sowohl steroidale als auch nichtsteroidale Verbindungen. Unter den kompetitiven Antiestrogenen sind neben den sogenannten reinen Antiestrogenen, wie z.B. 7α-[9-(4,4,5,5,5-Pentafluorpentylsulfinyl)-nonyl]-estra-1,3,5(10)-trien-3,17β-diol auch solche Verbindungen die neben ihrer antagonistischen auch beträchtliche agonistische, also estrogene, Wirkung besitzen. Prominentester Vertreter der letztgenannten ist das Tamoxifen.

Es ist bereits eine Vielzahl von Indikationen beschrieben, für die die Antiestrogene eingesetzt werden können. Bekanntestes Beispiel ist die seit langem praktizierte klinische Behandlung des Mamma-Carcinoms mit Tamoxifen.

Die Verwendung von Antiestrogenen (Centchroman) zur weiblichen Kontrazeption beim Menschen ist ebenfalls beschrieben (Nittyanand S, Kamboj VP [1992] Centchroman: contraceptive efficacy and safety profile. International Conference on Fertility Regulation, November 5-8, 1992 Bombay, India, programs and abstracts). Allerdings treten bei wirksamen Dosierungen unerwünschte Nebenwirkungen wie zum Beispiel osteoporetische Veränderungen auf, die auf die systemische Wirkung der Antioestrogene zurückzuführen sind. Die Estrogendeprivation, die nach einer Langzeitbehandlung mit einem Antiestrogen auftreten kann, limitiert zumindest deren regelmäßige Anwendung zur Kontrazeption bei der Frau.

Schließlich geht aus der DE-A 42 13 005 die Verwendung von Aromatasehemmern zur Empfängnisverhütung bei weiblichen Primaten im fortpflanzungsfähigen Alter in einer Dosierung, bei der der menstruelle Zyklus des weiblichen Primaten im wesentlichen unbeeinflußt bleibt, hervor. Die Absoluthöhe der für die kontrazeptive Wirkung erforderlichen Tagesdosen hängt dabei ganz von der Art des verwendeten Aromatasehemmers ab. Für hochaktive Aromatasehemmer liegen die Tagesdosen in der Regel zwischen etwa 0,05 bis etwa 30 mg. Bei weniger aktiven Aromatasehemmern können die Tagesdosen auch höher liegen.

Für eine Fertilitätskontrolle beim Mann stehen bis heute lediglich Kondome sowie eine Vasektomie zur Verfügung. Erstere sind sowohl von der Akzeptanz als auch von der kontrazeptiven Sicherheit nur bedingt geeignet, die Vasektomie ist im Hinblick auf die Fertilität in der Regel irreversibel. Ein hormonelles Kontrazeptivum, das den oralen Kontrazeptiva für die Frau hinsichtlich der Effektivität, der Sicherheit, der Art der Anwendung und der Akzeptanz vergleichbar wäre, ist bisher nicht in Sicht. Ein weiterer großer Vorteil der hormonalen Kontrazeption bei der Frau ist deren Reversibilität.

Eine Zusammenfassung des aktuellen Standes der Bemühungen zur Entwicklung eines Kontrazeptivums für den Mann findet sich bei U. F. Habenicht in "Sitzungsberichte der Gesellschaft Naturforschender Freunde zu Berlin", Band 31, 1991, S. 101-116.

Weder eine direkte Hemmung der Spermatogenese durch verschiedene Alkylantien oder das als "Chinapille" bekanntgewordene Gossypol noch eine indirekte Hemmung der Spermatogenese über eine Blockade des hypophysären-hypothalamischen Systems unter Verwendung von Testosteron-Derivaten, von LHRH-Analoga (Agonisten bzw. Antagonisten) in Kombination mit Testosteron-Derivaten oder mit einer Kombination eines Androgens mit einem Gestagen brachten bisher den gewünschten Erfolg.

Die beschriebenen Ansätze erfüllen letztendlich zumindest nicht eine der beiden wichtigsten Forderungen an ein modernes Kontrazeptivum für den Mann, nämlich der Forderung der Reversibilität der Methode und eines möglichst geringen Nebenwirkungspotentials.

Des weiteren wurde auch noch die Verwendung von Antigestagenen (kompetitive Progesteronrezeptorantagonisten) zur männlichen Fertilitätskontrolle beschrieben (DE-A 40 39 561.8).
Bei Behandlung männlicher Bonnet-Makaken mit RU 486 (11β-[(4-N,N-Dimethylamino)-phenyl]-17 β-hydroxy-17α-propinyl-4,9(10)-estradien-3-on) wurde eine Abnahme des Ejakulationsgewichtes, der Spermienanzahl pro Ejakulat, Abnahme der Beweglichkeit der Spermien, morphologische Anomalitäten der Spermien sowie ein Verlust/eine Beeinträchtigung der Akrosomen beobachtet.

Aufgabe der vorliegenden Erfindung ist die Bereitstellung eines Arzneimittels zur reversiblen Kontrolle der männlichen Fertilität, welches im Vergleich zu den bereits vorgeschlagenen Arzneimitteln für diese Indikation geringere Nebenwirkungen bzw. eine bessere Handhabbarkeit aufweisen soll.

Diese Aufgabe wird durch die Verwendung von steroidalen Antiestrogenen zur Herstellung von Arzneimitteln zur Kontrolle der männlichen Fertilität gelöst.

Es wurde nunmehr gefunden, daß Antiestrogene überraschenderweise den akrosomalen Status der Spermien verändern: So wird unter dem Einfluss von Antiestrogenen eine beginnende akrosomale Reaktion beobachtet. Gleichzeitig wird durch die Antiestrogene die Motilität der Spermien beeinträchtigt.
Die vorzeitige Induktion der akrosomalen Reaktion und die Einschränkung der Motilität der Spermien lassen darauf schließen, daß diese befruchtungsunfähig sind.

Auf der anderen Seite bleiben verschiedene Parameter der männlichen Sexualfunktionen durch die Antiestrogene unbeeinflußt: die Organgewichte des männlichen Reproduktionstraktes und die Spermienkonzentration werden nicht verändert.

Durch die Antiestrogene wird somit eine reversible Hemmung der Spermienfunktionen bewirkt, die für eine erfolgreiche Befruchtung essentiell sind.

Diese Ergebnisse sind umso erstaunlicher, als Antiestrogene wie z. B. Tamoxifen oder Clomiphen bei bestimmten männlichen Patienten zur Behebung von Fertilitätsstörungen eingesetzt wurden [Acosta et al., Fertil. Steril. 55, S. 1150-6, (1991)]. Dadurch soll einer in den Testes vermuteten lokalen erhöhten Estrogenkonzentration begegnet werden, die möglicherweise die Ursache für die Fertilitätsstörungen sein könnte. Bei diesen Patienten kommen zwei Wirkkomponenten der eingesetzten Antiestrogene zum Tragen: einmal die antiestrogene Wirkung per se, zum anderen die durch den Feedback-Mechanismus (Gegenregulation) bedingte endogene Testosteronerhöhung.

Die vorstehend beschriebenen Eigenschaften von Antiestrogenen ergeben sich aus Versuchen, die mit dem ICI-Antiestrogen 7α- [9-(4, 4,5, 5, 5-Pentafluorpentylsulfinyl)-nonyl]-estra-1, 3, 5 (10)-trien-3, 17β-diol an intakten adulten männlichen Ratten durchgeführt wurden.
Diese Verbindung kann als Standardverbindung für alle Verbindungen dieser Substanzklasse gelten.
Das Versuchsdesign und die Ergebnisse gehen aus der nachstehenden Aufstellung hervor:

| Ratten | intakte männliche Tiere, Gewicht ca. 200g |
|---|---|
| Mit ICI 7α- [9-(4, 4,5, 5, 5-Pentafluorpentylsulfinyl)-nonyl]-estra-1, 3, 5 (10)-trien-3, 17β-diol behandelte Gruppe (n= 6; Anzahl der Tiere) | 2,5 mg/kg in 0,2.ml Vehikel subcutan auf öliger Basis (Benzylbenzoat/Rhizinusöl) |
| Vehikelkontrolle (n = 6; Anzahl der Tiere) | 0,2 ml Vehikel 1x täglich |
| Behandlungsdauer | 28 Tage |
| Bestimmung der Organgewichte des männlichen Reproduktionstraktes und Histologie | Samenblase, Prostata, Hoden, Nebenhoden |
| Gewinnung der Spermien | Gewinnung aus dem Nebenhoden (epididymale Spermien) |
| Bestimmung von: | -Motilität |
| | -Anzahl |
| | -akrosomaler Status (entsprechend der WHO-Richlinien) |

Beobachtungen bezüglich der Organgewichte des männlichen Reproduktionstrakts und der Spermieneigenschaften:
1. Keine Effekte auf die Gewichte und die Histologie der untersuchten Organe
2. Hemmung der Motilität
3. Induktion einer vorzeitigen beginnenden akrosomalen Reaktion

Diese Beobachtungen zeigen eindeutig, daß Antiestrogene zur Herstellung von Arzneimitteln zur Kontrolle der männlichen Fertilität geeignet sind.

Als antiestrogen wirkende Verbindungen kommen erfindungsgemäß kompetitive Antiestrogene (Estrogenrezeptorantagonisten) infrage. Estrogenrezeptorantagonisten gemäß vorliegender Erfindung sind von Steroiden abgeleitete Verbindungen. Unter antiestrogen wirkenden Verbindungen, im weitesten Sinn, gemäß vorliegender Erfindung sollen nur solche Verbindungen verstanden werden, die möglichst selektiv wirken, d.h. die im wesentlichen nur die Wirkung von Estrogenen hemmen und/oder deren Konzentration senken.
Die Estrogenrezeptorantagonisten wirken kompetitiv, indem sie Estrogene vom Rezeptor verdrängen., Verbindungen vom Typ des Aminoglutethimids, d. h. in der 3-Stellung alkylierte 3-(4-Aminophenyl)piperidin-2,6-dione und andere, die außer auf den Estrogenspiegel auch auf andere Sexualhormonserumkonzentrationen erniedrigend wirken, sind gemäß vorliegender Erfindung als antiestrogen wirksame Verbindungen nicht geeignet.

Als steroidale Estrogenrezeptorantagonisten kommen beispielsweise infrage:
11α-Methoxy-17α-äthinyl-1,3,5(10)-östratrien-3,17β-diol und 16β-Äthylestradiol, N-n-Butyl-N-methyl-11-(3, 17β-dihydroxyestra-1,3,5(10)-trien- 7α-yl)-undecanamid und
7α-[9-(4,4,5,5,5-Pentafluorpentylsulfinyl)nonyl]estra-1,3,5(10)-trien-3, 17β-diol.

Diese Aufzählung ist nicht abschließend; auch andere in den genannten Veröffentlichungen beschriebenen Antiestrogene sowie solche aus hier nicht genannten Veröffentlichungen sind geeignet.

Die Antiestrogene können gemäß vorliegender Erfindung zur Unterdrückung der männlichen Fertilität nach unterschiedlichen Behandlungsschemata angewendet werden.

### 1. Intermittierende Behandlung

Einmalige tägliche bis wöchentliche orale Behandlung über 4 -12 Monate. Danach: behandlungsfreies Intervall von 3 -5 Monaten. Daran anschließend erneute Behandlung wie vorstehend.

### 2. Kontinuierliche Behandlung

Einmalige tägliche orale Applikation oder orale Applikation in zwei- bis maximal siebentätigen regelmäßigen Abständen oder Applikation von Depotformulierungen in regelmäßigen Abständen (z. B. 1x pro Monat, 1x pro Quartal usw.).

Für die Herstellung eines Arzneimittels zur Kontrolle der männlichen Fertilität werden die steroidalen Antiestrogene in einer mit einer täglichen Dosis von 0,1 bis 100 mg p.o. Tamoxifen [(Z)-2-[p-(1,2-Diphenyl-1-butenyl)-phenoxy]-N,N-dimethyl äthylamin] oder anderen Antiestrogenen {Nafoxidin: 1-2-[4-(6-Methoxy-2-phenyl-3,4-dihydro-1-naphthyl)-phenoxy]-äthylpyrrolidin Hydrochlorid; Raloxifen: 6-Hydroxy-2-(p-hydroxyphenyl)-benzo[b]thien-3-yl-p-(2- piperidino-ethoxyl)phenylketon Hydrochlorid; Mer 25: 1-[p-(2-Diäthylaminoäthoxy)-phenyl]-2-(p-methoxyphenyl) 1- phenyläthanol); Centchroman oder weitere Verbindungen vom 1,1,2-Triphenylbut-1-en-Typ, insbesondere das 1,1-Bis-(3'-acetoxyphenyl)-2-phenyl-but-1-en [J. CancerRes. Clin. Oncol., (1986), 112, S. 119-124} wirkequivalenten Menge eingesetzt.
Im Falle der Verwendung einer Depotformulierung zur Herstellung des erfindungsgemäßen Arzneimittels, wird diese Depotformulierung so gewählt, daß die tägliche Freisetzungsrate des steroidalen Antiestrogens 0,1 bis 100 mg Tamoxifen oder einer wirkequivalenten Menge eines anderen Antiestrogens entspricht.

Wirkequivalente Mengen anderer Antiestrogene, d. h. Mengen die für die Hemmung der männlichen Fertilität der angegebenen Tamoxifenmenge entsprechen, können beispielsweise im Uteruswachstumshemmtest nach Estrogenstimmulierung bestimmt werden.

Die Formulierung der Antiestrogene für die Zwecke der vorliegenden Erfindung erfolgt im Falle der Herstellung oraler Dosierungseinheiten vollkommen analog zur bereits bekannten Anwendung von Tamoxifen (Eur. J. Cancer Clin. Oncol, 1985,21,985 und J.S. Patterson, "10 Years of Tamoxifen in Breast Cancer" in HormonalManipulation of Cancer; Peptides, Growth Factors and New (Anti) steroidal Agents, Raven Press, New York (1987)).

Für die Anwendung des Antiestrogens in einer Depotformulierung kann es als Mikrokristallsuspension, als ölige Lösung oder in Form eines wirkstoffhaltigen Trägers (transdermales System) hergerichtet werden.

Die nachfolgenden Beispiele dienen der näheren Erläuterung der Erfindung.

### Beispiel 1

| | |
|---|---|
| 5,0 mg | 7α-[9-(4,4,5,5,5-Pentafluorpentylsulfinyl)nonyl]estra-1,3,5(10)-trien-3,17β-diol (reines Antiestrogen) |
| 150,0 mg | Lactose |
| 60,0 mg | Maisstärke |
| 2,5 mg | Poly-N- Vinylpyrrolidon 25 |
| 2,0 mg | Aerosil |
| 0.5 mg | Magnesiumstearat |
| 220,0 mg | Gesamtgewicht der Tablette, die in üblicher Weise auf einer Tablettenpresse hergestellt wird. Gegebenenfalls kann auch der erfindungsgemäße Wirkstoff mit jeweils der Hälfte der oben angegebenen Zusätze getrennt zu einer Zweischichtentablette gepreßt werden. |

## Patentansprüche

1. Verfahren zur Kontrolle der männlichen Fertilität, **dadurch gekennzeichnet, dass** ein Mittel enthaltend einen steroidalen Estrogenrezeptorantagonisten oral verabreicht wird.

2. Verfahren zur Kontrolle der männlichen Fertilität, **dadurch gekennzeichnet, dass** ein Mittel enthaltend einen steroidalen Estrogenrezeptorantagonisten in Form einer Depotformulierung verabreicht wird.

3. Verfahren nach Anspruch 2, **dadurch gekennzeichnet, dass** die Depotformulierung ein transdermales System ist.

4. Verfahren nach Anspruch 2, **dadurch gekennzeichnet, dass** die Depotformulierung eine ölige Lösung ist.

5. Verfahren nach Anspruch 2, **dadurch gekennzeichnet, dass** die Depotformulierung eine Mikrokristallsuspension ist.

6. Verfahren nach einem der vorherigen Ansprüche, **dadurch gekennzeichnet, dass** der steroidale Estrogenrezeptorantagonist 7α-[9-(4,4,5,5,5-Pentafluorpentylsulfinyl)-nonyl]estra-1,3,5(10)-trien-3,17β-diol ist.

## Claims

1. A method of controlling male fertility, **characterized in that** a composition comprising a steroidal estrogen receptor antagonist is administered orally.

2. A method of controlling male fertility, **characterized in that** a composition comprising a steroidal estrogen receptor antagonist is administered in the form of a depot formulation.

3. A method according to claim 2, **characterized in that** the depot formulation is a transdermal system.

4. The method according to claim 2, **characterized in that** the depot formulation is an oily solution.

5. The method according to claim 2, **characterized in that** the depot formulation is a microcrystal suspension.

6. The method according to any one of the preceding claims, **characterized in that** the steroidal estrogen receptor antagonist is 7α-[9-(4,4,5,5,5-pentafluoropentylsulfinyl)nonyl]estra-1,3,5(10)-triene-3,17β-diol.

## Revendications

1. Procédé pour la régulation de la fertilité masculine, **caractérisé en ce qu'**on administre par voie orale une composition contenant un antagoniste stéroïdien de récepteur d'oestrogène.

2. Procédé pour la régulation de la fertilité masculine, **caractérisé en ce qu'**on administre une composition contenant un antagoniste stéroïdien de récepteur d'oestrogène, sous forme d'une composition retard.

3. Procédé selon la revendication 2, **caractérisé en ce que** la composition retard est un système transdermique.

4. Procédé selon la revendication 2, **caractérisé en ce que** la composition retard est une solution huileuse.

5. Procédé selon la revendication 2, **caractérisé en ce que** la composition retard est une suspension de microcristaux.

6. Procédé selon l'une quelconque des revendications précédentes, **caractérisé en ce que** l'antagoniste de récepteur d'oestrogène est le 7α-[9-(4,4,5,5,5-pentafluoropentylsulfinyl)nonyl]estra-1,3,5(10)-triène-3,17β-diol.
